# EUROPEAN PATENT APPLICATION

(11) **EP 4 079 720 A1**
(43) Date of publication of application: **26.10.2022**
(21) Application number: 21169695.0
(22) Date of filing: 21.04.2021
(51) Int. Cl.: C07C 45/41, C07C 47/04, C07C 51/02, C07C 51/41, C07C 53/02, C07C 53/06, C01B 32/60, B01D 53/14, B01D 61/42

(54) **THE PRODUCTION OF FORMIC ACID OR FORMALDEHYDE FROM CARBON DIOXIDE**

(71) Applicant: Nederlandse Organisatie voor toegepast- natuurwetenschappelijk onderzoek TNO, 2595 DA 's-Gravenhage (NL)
(72) Inventor: The designation of the inventor has not yet been filed
(74) Representative: Nederlandsch Octrooibureau

(57) **Abstract**

The invention concerns a process and modular system for producing formic acid from a source of carbon dioxide. The process according to the invention comprises (a) a carbon capture step wherein a source of carbon dioxide is contacted with an alkaline solution to obtain a solution comprising carbonate and/or bicarbonate; optionally (b) subjecting the solution comprising carbonate and/or bicarbonate to alkaline water electrolysis, wherein carbonate present in the solution comprising carbonate and/or bicarbonate is converted to bicarbonate and H₂O is converted into H₂ and O₂; (c) subjecting the solution comprising carbonate and/or bicarbonate to a hydrogenation step in the presence of a catalyst to obtain a solution comprising formate; and (d) subjecting the solution comprising formate obtained in step (c) to bipolar membrane electrodialysis to obtain a concentrated formic acid solution and a recovered alkaline solution, wherein the recovered alkaline solution obtained in step (d) is recycled back to step (a). The concentrated formic acid solution obtained from step (d) may be subjected to a hydrogenation step in the presence of a hydrogenation catalyst to obtain a concentrated formaldehyde solution.

## Description

### Field of the invention

The present invention relates to a process for producing formic acid or formaldehyde and a modular system for performing the process, wherein the formic acid or formaldehyde are derived from a source of carbon dioxide.

### Background of the invention

Direct air capture of carbon dioxide gained attention in relation to the issue of global warming and the need for sustainable chemical building blocks. For the same reasons the use of carbon dioxide from flue gasses is investigated and applied. In direct air capture technology and the technologies for capturing flue gasses carbon dioxide is obtained in pure form. Subsequently carbon dioxide is stored or converted in chemical building blocks.

Other technologies convert carbon dioxide to a mixture of formate and formic acid. It is known from literature to produce formic acid in a process, wherein (pure) carbon dioxide is absorbed in an ammonia capture solvent, and subsequently converted to a formate salt in a hydrogenation reaction. Such a process is for example described in US 2016/137573. A disadvantage of US 2016/137573 is that hydrogenation does not have a high conversion rate to formic acid. Rather, mainly formate salts will be produced in such a process.

It is further known from US 2020/0038803 to regenerate carbon-rich amine solutions using bipolar membrane electrodialysis. US 2020/0038803 describes a regeneration system for a carbon-rich amine solution produced in carbon dioxide capture from a mixed gas, which is composed of a bipolar membrane electrodialysis apparatus and a carbon dioxide removal apparatus. The resulting product is pure carbon dioxide. US 2020/0038803 is silent on providing chemical building blocks such as formic acid.

In US 2007/254969, methanol is produced from pure carbon dioxide, for which a pure source of carbon dioxide is needed. The conversion occurs via electrochemical reduction of carbon dioxide in an electrolyser, which has a low productivity.

In EP 0597151, a method for preparing formic acid is disclosed, which employs a concentrated or pure source of carbon dioxide to be captured in a tertiary amine solution to form aminoformate, and subsequently an energy-intensive distillation of aminoformate to obtain formic acid.

There is a need for an efficient and durable process for the production of formic acid from carbon dioxide. There is need for such process which can use sources wherein the carbon dioxide is not in pure form and wherein there is no need to first concentrate carbon dioxide to its pure form. Such additional concentration step is inefficient. There is need for a process for the production of formic acid from carbon dioxide which can directly use sustainable electrical energy that is abundantly present such as electrical energy obtained from windmills and solar panels. There is need for such process wherein a concentrated formic acid solution without formate contamination can be obtained in an efficient way.

### Summary of the invention

The invention concerns a highly energy and cost efficient and durable process for the production of formic acid from carbon dioxide. In this process a carbon capture step is combined with a hydrogenation step to obtain formate and a bipolar membrane electrolysis step to provide a concentrated formic acid solution and to regenerate the alkaline capture liquid of the carbon capture step. The inventors have for the first time combined these steps in an integrated, efficient and durable process for the production of formic acid, which may in turn be further converted in formaldehyde.

The invention in particular concerns the following preferred embodiments:
1 A process for producing formic acid, comprising:
   (a) a carbon capture step wherein a source of carbon dioxide is contacted with an alkaline solution to obtain a solution comprising carbonate and/or bicarbonate;
   (c) subjecting the solution comprising carbonate and/or bicarbonate to a hydrogenation step in the presence of a catalyst to obtain a solution comprising formate; and
   (d) subjecting the solution comprising formate obtained in step (c) to bipolar membrane electrodialysis to obtain a concentrated formic acid solution and a recovered alkaline solution,
   wherein the recovered alkaline solution obtained in step (d) is recycled back to step (a).
2 The process according to embodiment 1, wherein the process is for producing formaldehyde and further comprises:
   (e) subjecting the concentrated formic acid solution obtained from step (d) to a hydrogenation step in the presence of a hydrogenation catalyst to obtain a concentrated formaldehyde solution.
3 The process according to embodiment 1 or 2 wherein the recovered alkaline solution comprises 1 - 20 wt.% formate on total weight, preferably 2 - 20 wt.% formate on total weight, more preferably 4 - 15 wt.% formate on total weight.
4 The process according to any one of the preceding embodiments, wherein in step (d) less than 80% of the formate of the solution comprising formate obtained in step (c) is converted to formic acid, preferably 10-70% of the formate of the solution comprising formate obtained in step (c) is converted to formic acid, more preferably 20-60% of the formate of the solution comprising formate obtained in step (c) is converted to formic acid, even more preferably 30-60% of the formate of the solution comprising formate obtained in step (c) is converted to formic acid.
5 The process according to any one of the preceding embodiments wherein the concentrated formic acid solution comprises less than 2 wt.% inorganic salts on formic acid weight, preferably less than 1 wt.% inorganic salts on formic acid weight, even more preferably the concentrated formic acid solution does not comprise inorganic salts.
6 The process according to any one of the preceding embodiments wherein the amount of carbon dioxide in the source of carbon dioxide is in the range of 50 - 200000 ppmv, preferably in the range of 100 - 150000 ppmv, even more preferably is in the range of 200 - 120000 ppmv.
7 The process according to any one of the preceding embodiments wherein the source of carbon dioxide is air and/or flue gas.
8 The process according to any one of the preceding embodiments, further comprising (b) subjecting the solution comprising carbonate and/or bicarbonate to alkaline water electrolysis prior to step (c), wherein carbonate present in the solution comprising carbonate and/or bicarbonate is converted to bicarbonate and H₂O is converted into H₂ and O₂, and wherein the H₂ formed is preferably provided as a H₂ source for the hydrogenation of step (c).
9 The process according to any one of the preceding embodiments, wherein H₂ gas required in step (c) and/or (e) originates from electrolysis of water.
10 The process according to any one of the preceding embodiments, wherein the alkaline solution is a solution comprising one or more selected from the list consisting of sodium hydroxide, potassium hydroxide, ammonium hydroxide, and lithium hydroxide, preferably wherein the alkaline solution comprises sodium hydroxide and/or potassium hydroxide.
11 A modular system for performing the process according to any one of embodiments 1 - 10, comprising:
   (a) an absorber having a first inlet for receiving an alkaline solution, a second inlet for receiving a source of carbon dioxide, an outlet for discharging a gas stream depleted in carbon dioxide, and an outlet for discharging a solution comprising carbonate and/or bicarbonate;
   (c) a hydrogenation reactor, comprising an inlet for receiving the solution comprising carbonate and/or bicarbonate, an inlet for receiving H₂ gas and an outlet to discharge a solution comprising formate; and
   (d) a bipolar membrane electrodialysis reactor, comprising an inlet for receiving the solution comprising formate, an inlet for receiving an electrolyte solution, an outlet for discharging the concentrated formic acid solution and an outlet for discharging a recovered alkaline solution,
   wherein the modular system comprises a recycle loop which connects the outlet for discharging the recovered alkaline solution to the inlet for receiving an alkaline solution.
12 The modular system according to embodiment 11, further comprising one or more modules selected from:
   (b) an alkaline water electrolysis reactor, comprising an inlet for receiving the solution comprising carbonate and/or bicarbonate, an outlet for discharging the solution comprising carbonate and/or bicarbonate bicarbonate and one or more outlets for discharging H₂ and O₂; and
   (e) a second hydrogenation reactor, comprising an inlet for receiving the concentrated formic acid solution from module (d), an inlet for receiving H₂ gas and an outlet for discharging formaldehyde.

### Detailed description of the invention

First and foremost, the invention concerns a process for the production of formic acid. In a second aspect, the invention concerns a system which is specifically designed for performing the process according to the invention. Here below, the process and system according to the invention are separately described. Nonetheless, the skilled person will appreciate that everything said for the process applies also the system and everything said for the system applies also for the process.

### The process for producing formic acid

In a first aspect, the invention concerns a process for the formation of formic acid from carbon dioxide. The process according the invention comprises:
(a) a carbon capture step wherein a source of carbon dioxide is contacted with an alkaline solution to obtain a solution comprising carbonate and/or bicarbonate;
(b) optionally subjecting the solution comprising carbonate and/or bicarbonate to alkaline water electrolysis prior to step (c), wherein carbonate present in the solution comprising carbonate and/or bicarbonate is converted to bicarbonate and H₂O is converted into H₂ and O₂;
(c) subjecting the solution comprising carbonate and/or bicarbonate to a hydrogenation step in the presence of a catalyst to obtain a solution comprising formate; and
(d) subjecting the solution comprising formate obtained in step (c) to bipolar membrane electrodialysis to obtain a concentrated formic acid solution and a recovered alkaline solution,
(e) optionally subjecting the concentrated formic acid solution obtained from step (d) to a hydrogenation step in the presence of a hydrogenation catalyst to obtain a concentrated formaldehyde solution,
wherein the recovered alkaline solution obtained in step (d) is recycled back to step (a).

### Step (a): Carbon capture

In step (a), the capture of carbon dioxide in an alkaline solution takes place. Such carbon capture is known in the art and typically takes place in a scrubber, also referred to as an absorber. The scrubber is fed with a source of carbon dioxide and an alkaline solution. In order to optimize contact between the alkaline solution and the carbon dioxide, the carbon dioxide is typically fed via a gas inlet at the bottom of the scrubber and the alkaline solution is fed via a liquid inlet at the top of the scrubber. During step (a), the carbon dioxide that is fed to the scrubber reacts with the alkaline compound present within the scrubber and forms a bicarbonate and/or carbonate salt. The formed bicarbonate and/or carbonate solution is typically discharged from the scrubber from a liquid outlet at the bottom of the scrubber. The absorber is preferably an absorption column, which is preferably operated in counter-current mode. Preferably, the absorber is selected from the group consisting of a tray column, a packed column, a spray column and a bubble column.

During step (a), carbon dioxide is transferred from the source of carbon dioxide to the alkaline solution. The alkaline solution is a solution comprising an alkaline compound and has a pH higher than 7.0. Due to the alkali nature of the alkaline solution upon transferal most or all of the carbon dioxide is converted to carbonate and/or bicarbonate. As is known in the art the equilibrium between carbon dioxide, carbonic acid, carbonate and bicarbonate is amongst others a function of the pH of the solution. Preferably the pH of the alkaline solution is above 8, more preferably above 8.5, even more preferably above 9.0. Preferably the pH of the alkaline solution is between 8.0 and 14.0, more preferably between 8.5 and 13.0, most preferably between 9.0 and 12.0. These range provide for optimal operation of the process. A higher pH improves the uptake of carbon dioxide in the absorber. However a very high pH is not desired as such pH imposes restrictions related to safety and material choice and therefore increases costs for the design and operation of the carbon capture step. Moreover, higher pH values may cause the equilibrium in the obtained solution between carbon dioxide (or carbonic acid), carbonate and bicarbonate to shift too much towards carbonate, which is undesirable for step (c). Thus, preferably as much as possible bicarbonate is produced in step (a). The amount of carbonate subjected to step (c) can be reduced by implementation of electrolysis step (b), which is preferably performed in such situations. Preferably, the solution comprising carbonate and/or bicarbonate comprises at least bicarbonate and typically it contains both carbonate and bicarbonate.

Preferably, in the carbon capture step more than 30 wt.% of the carbon dioxide of the source of carbon is transferred to the alkaline solution, more preferably more than 40 wt.% of the carbon dioxide of the source of carbon dioxide is transferred to the alkaline solution, even more preferably more than 45 wt.% of the carbon dioxide of the source of carbon dioxide is transferred to the alkaline solution. Preferably in the carbon capture step 30 - 95 wt.% of the carbon dioxide of the source of carbon dioxide is transferred to the alkaline solution, more preferably 40 - 80 wt.% of the carbon dioxide of the source of carbon dioxide is transferred to the alkaline solution, even more preferably 45 - 60 wt.% of the carbon dioxide of the source of carbon dioxide is transferred to the alkaline solution. The process is not limited by a maximal uptake of carbon dioxide, but from an efficiency point of view, a too high uptake would lead to increased investment and operational costs needed to capture the last bit of carbon dioxide from the source of carbon dioxide. Preferably, the sum of carbon dioxide, carbonic acid, carbonate and bicarbonate in the solution comprising carbonate and/or bicarbonate is above 0.01 mole per liter, more preferably above 0.1 mole per liter, most preferably in the range of 1 - 10 mole per liter. If needed, the amount of carbon dioxide can be calculated as carbon dioxide equivalent, wherein 1 mole of carbon dioxide, carbonic acid, carbonate and bicarbonate each count for 1 carbon dioxide equivalent and wherein the carbon dioxide equivalent can be expressed in grams of carbon dioxide. Preferably the solution comprising carbonate and/or bicarbonate comprises more than 1 wt.% on total weight of total carbon dioxide equivalent, even more preferably more than 5 wt.% on total weight of total carbon dioxide equivalent, most preferably more than 10 wt.% on total weight of total carbon dioxide equivalent.

The alkaline solution preferably comprises one or more alkaline compounds, preferably a hydroxide salt. The hydroxide salt is preferably selected from the list consisting of sodium hydroxide, potassium hydroxide, ammonium hydroxide and lithium hydroxide, more preferably wherein the alkaline solution comprises sodium hydroxide and/or potassium hydroxide. These bases are well-known alkali hydroxides used in alkaline solutions for carbon dioxide absorption. The alkaline solution may comprise mixtures of alkaline compounds. In addition, the alkaline solution may comprise formate. The inventors surprisingly found that the presence of formate in the alkaline solution does not affect the carbon dioxide uptake, while at the same time improves the overall process efficiency for the process for producing formic acid. Preferably, the alkaline solution comprises 1 - 20 wt.% formate on total weight, preferably 2 - 20 wt.% formate on total weight, more preferably 4 - 15 wt.% formate on total weight. It is particularly advantageous that the alkaline solution is at least in part recycled from step (d).

Contrary to most conventional processes in which carbon dioxide is captured, the source of carbon dioxide may be a dilute source of carbon dioxide, instead of a source of (substantially) pure carbon dioxide. The process is especially suitable for sources of carbon dioxide with low concentrations of carbon dioxide, such as flue gasses or even air. As the process does not rely on a source of pure carbon dioxide, it is highly versatile, especially in terms of location. Also, a specific additional pre-treatment to concentrate carbon dioxide is not necessary. When obtained from air, the carbon dioxide level of air directly can be lowered whilst at the same time producing a valuable product in the form of formic acid. The process is also especially suitable for industrial waste streams with relative low carbon dioxide levels such as flue gasses. Due to the relative low concentration the carbon dioxide of such streams, these typically are not utilized in carbon capture processes. The current invention enables the direct use for such streams; no further carbon dioxide concentration is necessary.

In one generally preferred embodiment the amount of carbon dioxide in the source of carbon dioxide is in the range of 50 - 200000 ppmv, preferably in the range of 100 - 150000 ppmv, even more preferably is in the range of 200 - 120000 ppmv. The unit ppmv refers to parts per million in volume. In terms of weight, it is preferred that the amount of carbon dioxide in the source of carbon dioxide is in the range of 0.01 -25 wt.% of total weight, more preferably in the range of 0.02 - 20 wt.% of total weight, even more preferably in the range of 0.02 - 15 wt.% of total weight, most preferably in the range of 0.03 - 15 wt.% of total weight. Herein, it is preferred that the source of carbon dioxide is air and/or flue gas.

In one specifically preferred embodiment the amount of carbon dioxide in the source of carbon dioxide is in the range of 50 - 5000 ppmv, preferably in the range of 100 - 1000 ppmv, even more preferably is in the range of 200 - 500 ppmv. In this embodiment preferably the amount of carbon dioxide in the source of carbon dioxide is in the range of 0.01 - 1.0 wt.% on total weight, more preferably in the range of 0.02 - 0.5 wt.% on total weight, even more preferably in the range of 0.02 - 0.1 wt.% on total weight, most preferably in the range of 0.04 - 0.08 wt.% on total weight. Herein, it is preferred that the source of carbon dioxide is air.

In another specifically preferred embodiment, the amount of carbon dioxide in the source of carbon dioxide is in the range of 1000 - 200000 ppmv, preferably in the range of 10000 - 150000 ppmv, even more preferably is in the range of 50000 - 120000 ppmv. In this embodiment preferably the amount of carbon dioxide in the source of carbon dioxide is in the range of 0.5 - 20 wt.% on total weight, more preferably in the range of 1 - 15 wt.% on total weight, even more preferably in the range of 2 - 20 wt.% on total weight, most preferably in the range of 5 - 15 wt.% on total weight. Herein, it is preferred that the source of the source of carbon dioxide is flue gas.

Step (a) further affords a gaseous product stream which is depleted in carbon dioxide with respect to the incoming source of carbon dioxide. This gaseous product can be used as deemed fit, such as emitted to the environment. The solution comprising carbonate and/or bicarbonate is subjected to the hydrogenation of step (c), optionally after an alkaline water electrolysis step (b).

### Step (b): Acidification

In a preferred embodiment, the pH is adjusted after step (a), if needed, to be in the range of 7.0 - 11.0, more preferably in the range of 7.5 - 10.0, most preferably in the range of 8.0 - 9.0. Of course, if the pH is already in this desired range, no adjustment is needed. Although step (c) can operate with pH values outside this range, the efficacy of this step is highly improved within this pH range. The inventors found that in case the concentration of carbon dioxide in the source of carbon dioxide is very low, the pH of the resulting solution, comprising carbonate and/or bicarbonate, obtained in step (a) is high, resulting in an equilibrium lying towards carbonate. This reduces the efficiency of the hydrogenation of step (c), wherein predominantly bicarbonate is converted to formate. In that case, the solution comprising carbonate and/or bicarbonate may be slightly acidified before it is subjected to step (c). This may be convenient in case the concentration of carbon dioxide in the source of carbon dioxide is below 5000 ppmv of carbon dioxide, preferably below 1000 ppmv of carbon dioxide, most preferably even below 500 ppmv of carbon dioxide and/or when the concentration of carbon dioxide in the source of carbon dioxide is below 1 wt.% of carbon dioxide on total weight, preferably below 0.5 wt.% of carbon dioxide on total weight, most preferably below 0.1 wt.% of carbon dioxide on total weight. In an especially preferred embodiment, the solution comprising carbonate and/or bicarbonate originating from step (a) is slightly acidified before it is subjected to step (c) in case the source of carbon dioxide is air.

In addition, the inventors realized that also for higher concentrations of carbon dioxide in the source of carbon dioxide, the pH of the solution comprising carbonate and/or bicarbonate is preferably optimized to provide an optimal feed for step (c). For example, considering a higher pH improves the uptake of carbon dioxide in step (a), a relative high pH of the alkaline solution is desirable during step (a). However, this may result in that the pH of the resulting solution comprising carbonate and/or bicarbonate is too high for optimal performance of step (c). Preferably, the solution comprising carbonate and/or bicarbonate obtained in step (a) is acidified before step (c). This is especially advantageous when the pH of the solution comprising carbonate and/or bicarbonate obtained in step (a) is above 11.0, preferably above 10.0, most preferably above 9.0.

The inventors have developed a highly efficient method for adjusting the pH (acidifying) of the solution comprising carbonate and/or bicarbonate originating from step (a). By subjecting the composition comprising carbonate and/or bicarbonate to alkaline water electrolysis prior to step (c), the pH can be lowered. At the same time, H₂ is generated that can be used in step (c) and/or (e) of the process according to the invention. Alkaline water electrolysis is known in the art, and is typically performed in an alkaline water electrolysis reactor comprising electrodes which are suitable for electrolysis of water into H₂ and O₂. The electrodes are separated by a membrane. On the anode side, O₂ and H⁺ are produced, while on the cathode side H₂ and OH⁻ are produced. By passing the solution comprising carbonate and/or bicarbonate on the anode side, the pH of the solution comprising carbonate and/or bicarbonate is lowered by the formation of H⁺. In other words, the carbonate is converted into bicarbonate by the formed H⁺ ions. At the same time, the alkaline water electrolysis provides H₂ at the cathode side that synergistically can be used as a source of H₂ for the hydrogenation of step (c) and/or step (e), preferably for step (c).

In case the solution comprising carbonate and/or bicarbonate contains some formate, the oxidation thereof can be minimized by selection of the appropriate catalyst at the anode and anode potential. The skilled person is aware of suitable materials and conditions to be used, e.g. from Shu etal., Environ. Sci. Technol. 2020, 54 (14), 8990-8998.

In a preferred embodiment the process for producing formic acid comprises step (b): subjecting the solution comprising carbonate and/or bicarbonate to alkaline water electrolysis prior to step (c), wherein carbonate present in the solution comprising carbonate and/or bicarbonate is converted into bicarbonate and H₂O is converted into H₂ and O₂, and wherein the H₂ formed is preferably provided as a H₂ source for the hydrogenation of step (c). In step (b), the solution comprising carbonate and/or bicarbonate obtained in step (a) is acidified. Preferably in step (b) the solution comprising carbonate and/or bicarbonate is fed on the anode side of the alkaline water electrolysis reactor.

### Step (c): Hydrogenation

In step (c) the solution comprising carbonate and/or bicarbonate is subjected to a hydrogenation step, wherein it is contacted with a catalyst in the presence of H₂ gas and wherein the carbonate and/or bicarbonate is converted into formate. Without being bound to a theory, it is expected that the bicarbonate is converted into formate by the catalyst, while at the same time the equilibrium between carbonate and bicarbonate shifts towards bicarbonate, which is then converted into formate. Such conversion of bicarbonate to formate is known in the art, e.g. from Su et al. ChemSusChem, 2015, 8(5), 813-816, and can be performed by the skilled person in any suitable way. Herein, a variety of catalysts based on amongst others ruthenium, iridium, rhodium, nickel, palladium, carbon, copper zinc oxide or iron may be utilized. Both heterogeneous and homogenous catalysts may be employed. Conversions of up to 95 % have been reported. In the context of the current invention, the catalyst is a hydrogenation catalyst, which is capable of catalysing a hydrogenation reaction. In a preferential embodiment the catalyst is a Ni/Ac, a Pd/C or a Pd/Ac catalyst (/C = on carbon; /Ac = on activated carbon). Step (c) is typically performed in a hydrogenation reactor employing conditions effective for converting carbonate and/or bicarbonate into formate.

To optimize the efficiency of step (c), the equilibrium between carbon dioxide, carbonic acid, carbonate and bicarbonate in the solution comprising carbonate and/or bicarbonate preferably is such that bicarbonate is the dominant species. In a preferred embodiment at least 60 mol% of the sum of carbon dioxide, carbonic acid, carbonate and bicarbonate of the solution comprising carbonate and/or bicarbonate is bicarbonate, when subjected to step (c), more preferably at least 80 mol%, most preferably at least 90 mol%. This equilibrium depends amongst others on the pH. Preferably, the pH of the solution comprising carbonate and/or bicarbonate in step (c) is in the range of 7.0 - 11.0, more preferably in the range of 7.5 - 10.0, most preferably in the range of 8.0 - 9.0.

In step (c), a solution comprising formate is obtained, also referred to herein as "hydrogenated solution". Preferably, the solution comprising formate comprises 4 - 30 wt.% formate on total weight, more preferably 5 - 25 wt.% formate on total weight, even more preferably 10 - 25 wt.% formate on total weight.

In the context of the present invention, any source of H₂ gas can be used in step (c). In light of the overall aim of providing a durable solution for the production of formic acid, it is preferred that the H₂ gas originates from a renewable source. In a preferred embodiment, the H₂ gas for step (c) originates at least in part from the electrolysis of water, preferably from the alkaline water electrolysis of step (b). The product of step (c) may contain unreacted H₂ gas. In one embodiment of the process according to the invention, this residual H₂ gas is separated from the solution comprising formate in step (f). Thus, it is preferred that the solution comprising formate originating from step (c) is subjected to step (f) to remove residual H₂ gas, preferably using a vapour-liquid separator, before it is subjected to electrodialysis of step (d), preferably wherein the removed H₂ gas is recycled to the hydrogenation of step (c). Such a H₂ separation step may be performed in a flash drum. Thus, in a preferred embodiment, the H₂ gas subjected to step (c) comprises a mixture of H₂ gas originating from the electrolysis of water of step (b) and a recycle from the downstream H₂ removal step (f).

### Step (d): Electrodialysis

The process for producing formic acid comprises step (d), wherein the solution comprising formate obtained in step (c) is subjected to bipolar membrane electrodialysis to obtain a concentrated formic acid solution and a recovered alkaline solution. In step (d), the solution comprising formate is fed to a bipolar membrane electrodialysis (BMED) cell and a concentrated formic acid solution and a recovered alkaline solution is obtained. BMED involves the dissociation of water in H⁺ and OH⁻ within the bipolar membrane (induced by the presence of an electrical field over the bipolar membrane) combined with electrodialysis wherein ionic species are transported from one solution to another by crossing one or more selectively permeable membranes. Thus, formate is removed from the solution of step (c) and protonated towards formic acid. Furthermore, the alkaline solution that is used in step (a) is recovered, and is preferably re-used in step (a). The inventors surprisingly found that BMED is excellent for obtaining a highly concentrated stream of formic acid that is not contaminated with formate from a solution comprising formate, and at the same time is excellent for recovering an alkaline solution that can be re-used in the carbon capture step (a).

Step (d) can be performed in any BMED reactor known in the art. A BMED reactor typically comprises one or more cells. Typically, each cell comprises two bipolar membranes on the outside of the cell and one ortwo permeable membranes within the cell, forming two orthree compartments. The diluate stream enters and exits one compartment and during its residence in the compartment, ionic species are transferred over the selectively permeable membrane towards one or two concentrate compartments. If one selective permeable membrane is present, the BMED cell consists of two compartments while the presence of two selective permeable membranes gives a three-compartment BMED cell. A typical arrangement of a BMED cell includes a diluate compartment and a concentrate compartment, which are separated by a cation or anion exchange membrane. Rinse compartments are located between the diluate compartment and the cathode and between the concentrate compartment and the anode. In a three compartment BMED, there is also a middle compartment facing two selective permeable membranes. Bipolar membrane electrodialysis is known in the art and the application for converting formate to formic acid is known, for example from the Journal of Membrane Science 325 (2008) page 528-536 and in the Journal of Membrane Science 328 (2009) pages 75-80.

Typically, a rinsing solution is fed to the cathode compartment to produce H₂ and OH⁻, and a rinsing solution is fed to the anode compartment to product O₂ and H⁺. Typically, the rinsing solution fed to both rinsing compartments is the same. The formate containing stream is fed into the diluate compartment, while formate is transferred through the anion exchange membrane to the concentrate compartment. At the same time, the diluate compartment is enriched with OH⁻ from the bipolar membrane. The concentrate stream may be a loop wherein the concentration of formate increases. The loop typically contains a bleed stream, as a branch to collect formic acid from the concentrate loop, as well as a feed stream to replenish the concentrate loop with water and polyelectrolytes. The bleed stream comprising formic acid is preferably subjected to the hydrogenation of step (e).

In exceptional circumstance, some bicarbonate ions may cross the anion exchange membrane from the diluate to the concentrate compartment, where they will recombine with H⁺ to form CO₂. In a preferred embodiment, the thus formed CO₂ is stripped from the BMED reactor. Such CO₂ stripping is known in the art.

Preferably, the solution comprising formate is fed to the diluate compartment of a two-compartment BMED cell and the diluate compartment is separated from the concentrate compartment by an anion exchange membrane. The solution comprising formate may also be fed to the concentrate compartment of a two-compartment BMED cell and the diluate compartment is separated from the concentrate compartment by a cation exchange membrane, although this is less preferred. The preferred configuration surprisingly gives a much lower formate contamination in the concentrated formic acid solution. This provides a high quality concentrated formic acid solution which subsequently provides high quality formaldehyde.

In a preferred embodiment, the BMED of step (d) is two-compartment BMED. Compared to three-compartment BMED, two-compartment BMED is more favorable in terms of energy consumption and has a more straightforward and therefore cheaper design. Thus, it is especially preferred that the solution comprising formate is fed to the diluate compartment of a two-compartment BMED cell and the diluate compartment is separated from the concentrate compartment by an anion exchange membrane. It is also possible to feed the solution comprising formate to the concentrate compartment of a two-compartment BMED cell and the diluate compartment is separated from the concentrate compartment by a cation exchange membrane.

During electrodialysis, the solution comprising formate is deprived of formate whilst at the same time the pH of the solution is increased by the formation of OH⁻ at the bipolar membrane facing the diluate compartment, resulting in recovery of the alkaline solution. In the concentrate compartment, formate is accumulated that is converted in formic acid by the H⁺ that is formed at the bipolar membrane facing the concentrate compartment.

The inventors surprisingly found that the BMED of step (d) does not need to be operated at the highest possible yield to have an efficient process. BMED processes in which substantially all of the formate entering the reactor is converted into formic acid, to provide pure formic acid and hydroxide solutions were found to be energetically challenging. The inventors surprisingly found that a relative low yield of formic acid in step (d) is preferred, as it considerably lowers the energy use of the BMED whilst not affecting the overall process yield. The relative low conversion into formic acid during step (d) gives a recovered alkaline solution containing some formate still, which was found not to jeopardize the overall process efficiency. Specifically the uptake of carbon dioxide in step (a) is not affected, nor is the effectiveness of the optional alkaline water electrolysis of step (b) and of the hydrogenation step (c). Any unconverted formate will thus be fed again to step (d), wherein it may be converted into formic acid. As the formic acid product stream will not contain substantial amounts of formate, the end product and overall process yield are not negatively affected by incomplete conversion of formate into formic acid during step (d).

In a preferred embodiment, the recovered alkaline solution comprises 1 - 20 wt.% formate on total weight, more preferably 2 - 20 wt.% formate on total weight, even more preferably 4 - 15 wt.% formate on total weight. Preferably, in step (d) less than 80 % of the formate of the solution comprising formate obtained in step (c) is converted to formic acid, more preferably 10 - 70 % of the formate of the solution comprising formate obtained in step (c) is converted to formic acid, more preferably 20 - 60 % of the formate of the solution comprising formate obtained in step (c) is converted to formic acid, even more preferably 30- 60 % of the formate of the solution comprising formate obtained in step (c) is converted to formic acid.

The concentrated formic acid solution obtained in step (d) comprises a high amount of formic acid. Preferably the concentrated formic acid solution comprises 5 wt.% or more formic acid, even more preferably 8 wt.% or more formic acid, most preferably 10 wt.% or more formic acid, based on total weight. Alternatively, the concentrated formic acid solution preferably is a solution of 0.5 - 3 M formic acid in water, preferably 1.5 - 2.5 M formic acid in water. Such a high concentration is advantageous for isolation of the formic acid and/or for further processing of the concentrated formic acid solution into for example formaldehyde. Step (d) affords a concentrated formic acid solution without substantial amounts of formate, such as less than 1 wt.% on total formic acid, even more preferably less than 0.5 wt.% on total formic acid, most preferably less than 0.1 wt.% on total formic acid. Overall, the purity of formic acid (i.e. formic acid content on dry weight) in the concentrated formic acid solution obtained in step (d) may be as high as 75 - 100 wt.%, preferably 80 - 95 wt.%.

The formic acid stream may contain electrolyte that is fed to the BMED cell. This electrolyte enters the BMED reactor comprised in the electrolyte solution. Such electrolyte may be selected from inorganic salts, polyelectrolytes and ion exchange resins, preferably from polyelectrolytes and ion exchange resins, most preferably the electrolyte is a polyelectrolyte. Suitable inorganic salts include sodium hydroxide, potassium hydroxide, ammonium hydroxide, calcium hydroxide. Suitable polyelectrolytes include polyionenes, polyacrylic acids, polyethyleneimines, polyacrylamides, polystyrene sulfonates, polyallylamines, carboxymethyl celluloses, polygalacturonic acids, alginic acids, polypeptides, as well as salts, derivatives, copolymers and combinations thereof. Preferred polyelectrolytes include poly(sodium-4-styrenesulfonate), poly(acrylic acid-co-maleic acid), poly(allylamine hydrochloride), poly(diallyldimethyl ammonium chloride), sodium polyacrylate and sodium poly(acrylamide-2-methyl-1-propanesulfoate). Commercially available polyelectrolytes that may also be suitable include but are not limited to Duramax^{™}, Tamol^{™}, Romax^{™} and Dowex^{®} (Dow Chemical), Acusol^{™} and Acumer^{™} (Rohm and Haas), Dispex^{®} and Magnafloc^{®} (BASF) and Rheosiove^{™} and Terrablend^{™} (Arkema). Suitable ion exchange resins include Amberlite IR120, Nafion^{®} PFSA Superacid Resins NR-40 and Nafion^{®} PFSA Superacid Resins NR-50. Polyacids are especially preferred electrolytes. In a preferred embodiment the electrolyte solution comprises formic acid, to have a slightly acidic pH. The formic acid fed via the electrolyte solution ends up in the concentrated formic acid solution, so does not jeopardize the overall working of the process according to the invention. Preferably, the electrolyte solution comprises 0.1 - 5 wt.% formic acid on total weight.

Even though some electrolyte may end up in the concentrated formic acid solution comprises, it is preferred that it comprises less than 2 wt.% inorganic salts, based on formic acid weight, preferably less than 1 wt.%, more preferably less than 0.5 wt.%, most preferably less than 0.1 wt.% inorganic salts, based on formic acid weight.

### Step (e): Hydrogenation to formaldehyde

In an especially preferred embodiment, the formic acid obtained in step (d) is concerted into formaldehyde. Formaldehyde is a valuable feedstock for the petrochemical industry and serves as a building block for fuels and for more complex chemicals. The process according to the present embodiment may also be referred to as being for producing formaldehyde. In step (e), the concentrated formic acid solution is subjected to a hydrogenation step in the presence of a hydrogenation catalyst to obtain a formaldehyde. Suitable catalysts are known in the art, and include a Pd/Ac catalyst. The concentrated formic acid solution is contacted with a catalyst in the presence of H₂ gas, to obtain a concentrated formaldehyde solution. Such conversion of formic acid to formaldehyde is known in the art (see e.g. NASA, GARD Project 1416, Research and development study related to the synthesis of formaldehyde from CO2 and H2, quarterly progress report No. 1, 1966, available via https://ntrs.nasa.gov/citations/19670012844) and can be performed by the skilled person in any suitable way. As will be understood by the skilled person, step (e) is performed in the presence of a hydrogenation catalyst and employs conditions suitable for converting formic acid into formaldehyde.

In the context of the present invention, any source of H₂ gas can be used in step (e). In light of the overall aim of providing a durable solution for the production of formic acid, it is preferred that the H₂ gas originates from a renewable source. In a preferred embodiment, the H₂ gas for step (e) originates from electrolysis of water, such as the electrolysis of step (b). The product of step (e) may contain unreacted H₂ gas. In one embodiment of the process according to the invention, this residual H₂ gas is separated from the concentrated formaldehyde solution formed in step (e). Thus, it is preferred that the concentrated formaldehyde solution originating from step (e) is subjected to step (g) to remove residual H₂ gas, preferably using a vapour-liquid separator. Preferably, the H₂ gas is recycled to the hydrogenation of step (e). Such a H₂ separation step may be performed in a flash drum. Thus, in a preferred embodiment, the H₂ gas subjected to step (e) comprises a mixture of H₂ gas originating from the electrolysis of water, such as from step (b), and a recycle from the downstream H₂ removal step (g).

### The modular system for the process for producing formic acid

In a second aspect, the invention concerns a modular system for the process for producing formic acid. The modular system comprises:
(a) an absorber having a first inlet for receiving an alkaline solution, a second inlet for receiving a source of carbon dioxide, an outlet for discharging a gas stream depleted in carbon dioxide, and an outlet for discharging a solution comprising carbonate and/or bicarbonate;
(c) a hydrogenation reactor, comprising an inlet for receiving the solution comprising carbonate and/or bicarbonate, an inlet for receiving H₂ gas and an outlet to discharge a solution comprising formate; and
(d) a bipolar membrane electrodialysis (BMED) reactor, comprising an inlet for receiving the solution comprising formate, an inlet for receiving an electrolyte solution, an outlet for discharging the concentrated formic acid solution and an outlet for discharging a recovered alkaline solution,
wherein the modular system comprises a recycle loop which connects the outlet for discharging the recovered alkaline solution to the inlet for receiving an alkaline solution.

Preferably, the system further comprises one or more of the following modules:
(b) an alkaline water electrolysis module, comprising a cathode and an anode, an inlet for receiving the solution comprising carbonate and/or bicarbonate from module (a) at the anode side, an inlet for receiving water at the cathode side, an outlet for discharging an acidified solution comprising carbonate and/or bicarbonate and one or more outlets for discharging H₂ and O₂; and
(e) a second hydrogenation reactor, comprising an inlet for receiving the concentrated formic acid solution, an inlet for receiving H₂ gas and an outlet to discharge a solution comprising formaldehyde.
Herein, the acidified solution comprising carbonate and/or bicarbonate is used as feed for module (c).

Module (a) is for performing step (a), module (b) is for performing step (b), module (c) is for performing step (c), module (d) is for performing step (d), and module (e) is for performing step (e). The modules of the system according to the invention are interconnected to allow fluid connectivity of the streams between the modules. Such fluid connectivities exist between the outlet of one module and the inlet of the subsequent module.

Carbon capture modules are known in the art, and any suitable carbon capture module can be used as module (a). It comprises a scrubber having a first inlet for receiving an alkaline solution, a second inlet for receiving a source of carbon dioxide, a gas outlet in the top part of the scrubber and an outlet in the bottom part of the scrubber for discharging a solution comprising carbonate and/or bicarbonate.

Hydrogenation reactors are known in the art, and any suitable hydrogenation reactor can be used as module (c). It comprises an inlet for receiving the solution comprising carbonate and/or bicarbonate from module (a) or (b), an inlet for receiving H₂ gas, and an outlet to discharge a formate solution. The hydrogenation reactor comprises a catalyst capable of performing a hydrogenation reaction to convert bicarbonate into formate.

Bipolar membrane electrodialysis (BMED) reactor are known in the art, and any suitable bipolar membrane electrodialysis reactor can be used as module (d). It comprises a first inlet for receiving the solution comprising formate, a second inlet for receiving an electrolyte solution, a first outlet for discharging the concentrated formic acid solution and a second outlet for discharging a recovered alkaline solution. The second outlet is in fluid connection with the first inlet of carbon capture module (a). Preferably, the first outlet is is in fluid connection with the first inlet of second hydrogenation reactor (e). Bipolar membrane electrodialysis reactor (d) contains one or more electrochemical cells, each comprising a diluate compartment containing a cathode, a concentrate compartment comprising an anode and a cation or anion exchange membrane separating these compartments. Each cell further comprises two bipolar membranes on the outside of the cell. Preferably, the bipolar membrane electrodialysis reactor is a two-compartment BMED cell, and comprises one membrane separating the two compartments. That membrane may be a cation exchange membrane or an anion exchange membrane, preferably it is an anion exchange membrane. Typically, the BMED cell contains a two rinse compartments, located between the cathode and the diluate compartment and between the anode and the concentrate compartment. These rinse compartments are each equipped with a separate inlet for receiving a rinse solution and an outlet for discharging the rinse solution, and further contain outlets for discharging hydrogen gas or oxygen gas.

The system according to the invention may further comprise an alkaline water electrolysis module (b). Alkaline water electrolysis module are known in the art, and any suitable alkaline water electrolysis module can be used as module (b). It contains one or more electrochemical cells, each containing an anode and a cathode, which may be separated be a membrane to form an anode compartment and a cathode compartment. It further comprises an inlet for receiving the solution comprising carbonate and/or bicarbonate from module (a) and an outlet for discharging an acidified solution comprising carbonate and/or bicarbonate, that is to be used as solution comprising carbonate and/or bicarbonate in module (c). Module (b) typically further comprises an inlet for receiving water at the cathode side and gas outlets for discharging oxygen gas at the anode and H₂ gas at the cathode, preferably wherein the outlet for discharging H₂ gas is in fluid connection with the gas inlet of module (c) or (e), preferably module (c).

The system according to the invention may further comprise a second hydrogenation reactor (e), comprising a first inlet for receiving the formic acid solution from module (d), a second for receiving H₂ gas, and an outlet for discharging formaldehyde. Hydrogenation reactors are known in the art, and any suitable hydrogenation reactor can be used as module (e).

### General Definitions

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". The word "about" or "approximately" when used in association with a numerical value (e.g. about 10) preferably means that the value may be the given value more or less 1% of the value.

The present invention has been described above with reference to a number of exemplary embodiments. Modifications and alternative implementations of some parts or elements are possible, and are included in the scope of protection as defined in the appended claims. All citations of literature and patent documents are hereby incorporated by reference.

### Description of the drawings

The present invention is illustrated with two preferred embodiments depicted in the figures.

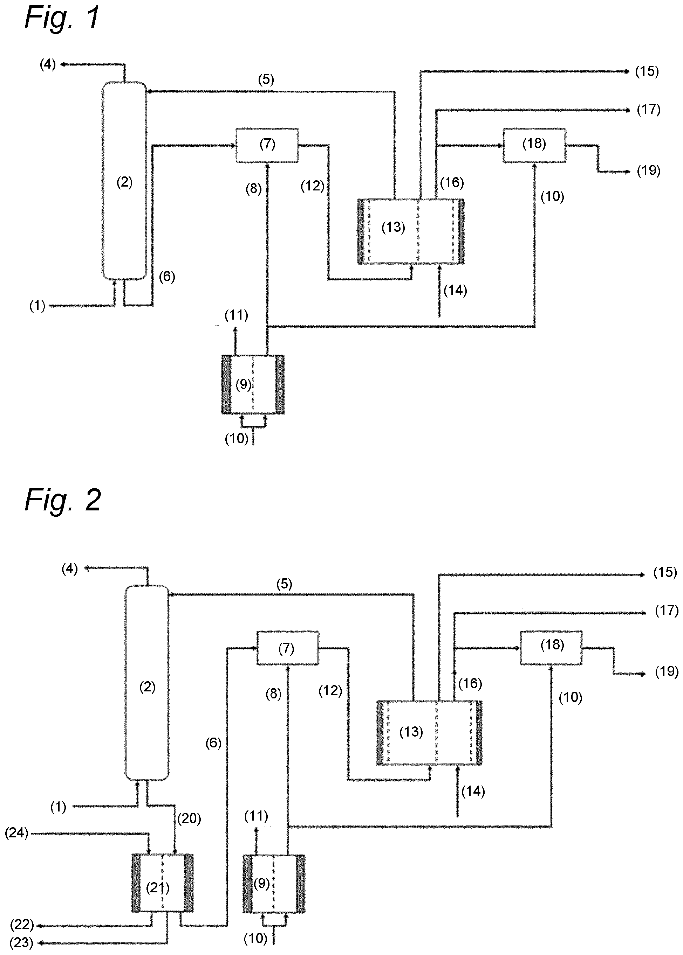

Figure 1 depicts a process flow diagram of the process according to the invention, in which flue gas is the source of carbon dioxide, typically with a CO₂ content in the range of 0.04 - 20 wt.%. Flue gas (1) and an alkaline solution (5) enter absorber (2), wherein absorption of CO₂ into the solution of step (a) takes place. Typically, over 50 % of the CO₂ present in the source of carbon dioxide is captured during step (a). A gaseous stream depleted in carbon dioxide (4) is discharged from the top of the absorber, while a solution comprising carbonate and/or bicarbonate (6) is discharged from the bottom. Solution (6) is fed to hydrogenation reactor (7), together with H₂ gas (8) which is typically generated in water electrolyzer (9), which is fed with water (10) and in addition to H₂ gas also generates O₂ gas (11). In hydrogenation reactor (7), step (c) occurs, which typically operates with a yield of over 80 %, and the carbonate and/or bicarbonate are hydrogenated into formate (12). The resulting solution comprising formate (12) that is obtained from reactor (7) is fed to BMED reactor (13). A second liquid (14), typically an electrolyte solution, enters the BMED reactor (13). Step (d) occurs in BMED reactor (13), wherein formate is converted into formic acid, and a recovered alkaline solution, which is depleted in formate (5), which usually has a pH of above 9.5, is fed back to absorber (2) as the alkaline solution. A concentrated formic acid solution (16) leaves the BMED reactor (13), together with some stripped carbon dioxide (15). The thus formed formic acid may be the product (17) of the process, or may be fed to hydrogenation reactor (18) to perform step (e). H₂ gas (10) from water electrolyzer (9) is fed to reactor (18), and formic acid is converted into formaldehyde (19), which typically operates with a yield of over 80 %. The resulting concentrated formaldehyde stream (19) is discharged from reactor (18).

Figure 2 depicts a process flow diagram of the process according to the invention, in which air is the source of carbon dioxide (direct air capture). Air (1) and an alkaline solution (5) enter absorber (2), wherein absorption of CO₂ into the solution of step (a) takes place. Typically, about 50 % of the CO₂ of the source of carbon dioxide is captured during step (a). A gaseous stream depleted in carbon dioxide (4) is discharged from the top of the absorber, while a solution comprising carbonate and/or bicarbonate (20) is discharged from the bottom. Solution (20) is subjected to alkaline water electrolysis in reactor (21). A KOH solution (24) is fed into reactor (21), and electrolysis of water of step (b) affords gaseous H₂ (22) and O₂ (23), that are discharged from the electrodes. The acidified solution comprising carbonate and/or bicarbonate (6) is discharged from reactor (21). The H₂ gas (22) formed in reactor (21) may be fed to hydrogenation reactor (7) and/or (18). Alternatively or additionally, additional H₂ gas (8) may be generated in water electrolyzer (9) and fed to hydrogenation reactor (7) and/or (18). The acidified solution (6) obtained from reactor (21) is fed to hydrogenation reactor (7), together with H₂ gas (8) which is typically generated in water electrolyzer (9), which is fed with water (10) and in addition to H₂ gas also generates O₂ gas (11). In hydrogenation reactor (7), step (c) occurs, which typically operates with a yield of over 80 %, and the carbonate and/or bicarbonate are hydrogenated into formate (12). The resulting solution comprising formate (12) that is obtained from reactor (7) is fed to BMED reactor (13). A second liquid (14), typically an electrolyte solution, enters the BMED reactor (13). Step (d) occurs in BMED reactor (13), wherein formate is converted into formic acid, and a recovered alkaline solution, which is depleted in formate (5), which usually has a pH of above 9.5, is fed back to absorber (2) as the alkaline solution. A concentrated formic acid solution (16) leaves the BMED reactor (13), together with some stripped carbon dioxide (15). The thus formed formic acid may be the product (17) of the process, or may be fed to hydrogenation reactor (18) to perform step (e). H₂ gas (10) from water electrolyzer (9) is fed to reactor (18), and formic acid is converted into formaldehyde (19), which typically operates with a yield of over 80 %. The resulting concentrated formaldehyde stream (19) is discharged from reactor (18).

### Examples

The modelling approach for a bipolar membrane electrodialysis for separation and protonation of formate to formic acid is according to the publications of Jaime Ferrer et al. (Jaime-Ferrer et al., J. Memb. Sci. 2006, 280 (1-2), 509-516; Jaime-Ferrer et al., J. Memb. Sci. 2008, 325 (2), 528-536; Jaime-Ferrer et al., Modelling. J. Memb. Sci. 2009, 328 (1-2), 75-80). The results presented in this example are based on a 2-compartment bipolar membrane electrodialysis cells. The cells comprise a diluate compartment, which is the cathode compartment to which the solution comprising formate is introduced, and a concentrate compartment, which is the anode compartment to which the electrolyte solution is fed. The compartments are separated by an anion exchange membrane. Each compartment is bordered by a bipolar membrane. Concentration curves for all species in solution in the two compartments are provided as a function of processing time. The model produced the volume variations per compartment as a function of time, and the differential current efficiency as a function of time, which is related to the overall efficiency of the process for formic acid production from formate.

The governing mechanisms in the bipolar membrane electrodialysis process are the alkalinization of the diluate compartment and the concomitant acidification of the concentrate compartment due to the action of the bipolar membranes. The alkalinization and acidification fluxes, which are equivalent to the water splitting flux at the bipolar membrane, is regulated by the applied electric current. The water splitting flux determines the migration of the different ions in the electrodialysis process. When one molecule of water is split into hydroxide anions towards the diluate compartment, and hydronium cations towards the concentrate compartment, one anion molecule crosses the anion exchange membrane from diluate to concentrate. The crossover of cations through the bipolar membrane (sodium, potassium, etc.) due to migration hinders the water splitting action in the bipolar membrane.

Other modelled phenomena in the bipolar membrane electrodialysis process are the mass transfer of formic acid through the bipolar membranes and the anion exchange membrane due to concentration gradient between compartments, the migration of monovalent ions in the direction of the electric field as a function of both the concentration in the compartment from which the ion migrates, and the applied electric current; the electro-osmotic effect of solvent transfer through anion exchange membrane from diluate to concentrate compartments, the homogeneous reactions coming from the buffer equilibria of formate - formic acid and solubilized carbon dioxide - bicarbonate - carbonate, and water auto-ionization, and the carbon dioxide desorption at the concentrate compartment when a carrier gas is bubbled at the bottom of an hypothetical buffer vessel in which the concentrate compartment stream is recirculated. The model equations for these phenomena can all be found in the above publications of Jaime Ferrer et al.

The main assumptions of the model are the following: a constant current density is applied throughout the whole process, with no effect on the total cell potential; the model considers an infinite set of cells, diluate and concentrate compartments, being both compartments separated by an anion exchange membranes, and each cell separated from each other by a bipolar membrane, thus meaning that the electrode compartments have not been taken into account; the anion transfer rate for bicarbonate and formate through the anion exchange membranes is taken to be the same for both; only monovalent anions are transferred through the different ion exchange membranes, thus meaning specifically that carbonate does not cross any of the membranes; constant temperature has been taken throughout the whole process.

All necessary parameters for the mathematical model, as mass transfer coefficients of the different species in solution through ion exchange membranes, the migration terms for charged species through ion exchange membranes, and the parameters for the electro-osmotic effect of volume transfer between different compartments, have been taken from Jaime Ferrer et al.. When data was not available for a specific parameter, reasonable assumptions were made based on presented data in Jaime Ferrer et al.

The described model can predict the loss of efficiency in the bipolar membrane electrodialysis process as the conversion of formate to formic acid proceeds. In Table 1, the different values of Differential Current Efficiency as a function of the conversion of formate present at the diluate compartment to formic at the concentrate compartment. The Differential Current Efficiency is defined as the quotient between the formic acid transfer rate at the concentrate compartment and the total acidification rate at the bipolar membrane, which is the driving force of the bipolar membrane electrodialysis process. The initial conditions for the results in table 1 are: initial concentrations of bicarbonate, carbonate and formate at the diluate compartment are 250, 75 and 3000 mol·m⁻³, respectively; initial concentrations of bicarbonate, carbonate and formic acid at the concentrate compartment are 0, 0 and 10 mol·m⁻³, respectively; constant current density applied is 250 A·m⁻², total membrane area of the bipolar membrane electrodialysis cell is 0.02 m²; initial volume of the diluate and concentrate compartment solutions is 10⁻⁴ and 10⁻⁴ m³, respectively.

**Table 1. Results of the model for the Differential Current Efficiency for formic acid removal as a function of the formate to formic acid conversion.**

| **Conversion formate to formic acid** [-] | **Differential Current Efficiency** [-] |
|---|---|
| 10% | 94% |
| 20% | 88% |
| 30% | 83% |
| 40% | 78% |
| 50% | 72% |
| 60% | 66% |

The simulation as represented in table 1 demonstrate a linear decrease of differential current efficiency upon a higher conversion of formate to formic acid. A loss of differential current efficiency directly relates to a higher energy usage and to a less efficient process.

## Claims

1. A process for producing formic acid, comprising:
(a) a carbon capture step wherein a source of carbon dioxide is contacted with an alkaline solution to obtain a solution comprising carbonate and/or bicarbonate;
(c) subjecting the solution comprising carbonate and/or bicarbonate to a hydrogenation step in the presence of a catalyst to obtain a solution comprising formate; and
(d) subjecting the solution comprising formate obtained in step (c) to bipolar membrane electrodialysis to obtain a concentrated formic acid solution and a recovered alkaline solution,
wherein the recovered alkaline solution obtained in step (d) is recycled back to step (a).

2. The process according to claim 1, wherein the process is for producing formaldehyde and further comprises:
(e) subjecting the concentrated formic acid solution obtained from step (d) to a hydrogenation step in the presence of a hydrogenation catalyst to obtain a concentrated formaldehyde solution.

3. The process according to claim 1 or 2 wherein the recovered alkaline solution comprises 1 - 20 wt.% formate on total weight, preferably 2 - 20 wt.% formate on total weight, more preferably 4 - 15 wt.% formate on total weight.

4. The process according to any one of the preceding claims, wherein in step (d) less than 80% of the formate of the solution comprising formate obtained in step (c) is converted to formic acid, preferably 10-70% of the formate of the solution comprising formate obtained in step (c) is converted to formic acid, more preferably 20-60% of the formate of the solution comprising formate obtained in step (c) is converted to formic acid, even more preferably 30-60% of the formate of the solution comprising formate obtained in step (c) is converted to formic acid.

5. The process according to any one of the preceding claims wherein the concentrated formic acid solution comprises less than 2 wt.% inorganic salts on formic acid weight, preferably less than 1 wt.% inorganic salts on formic acid weight, even more preferably the concentrated formic acid solution does not comprise inorganic salts.

6. The process according to any one of the preceding claims wherein the amount of carbon dioxide in the source of carbon dioxide is in the range of 50 - 200000 ppmv, preferably in the range of 100 - 150000 ppmv, even more preferably is in the range of 200 - 120000 ppmv.

7. The process according to any one of the preceding claims wherein the source of carbon dioxide is air and/or flue gas.

8. The process according to any one of the preceding claims, further comprising (b) subjecting the solution comprising carbonate and/or bicarbonate to alkaline water electrolysis prior to step (c), wherein carbonate present in the solution comprising carbonate and/or bicarbonate is converted to bicarbonate and H₂O is converted into H₂ and O₂, and wherein the H₂ formed is preferably provided as a H₂ source for the hydrogenation of step (c).

9. The process according to any one of the preceding claims, wherein H₂ gas required in step (c) and/or (e) originates from electrolysis of water.

10. The process according to any one of the preceding claims, wherein the alkaline solution is a solution comprising one or more selected from the list consisting of sodium hydroxide, potassium hydroxide, ammonium hydroxide, and lithium hydroxide, preferably wherein the alkaline solution comprises sodium hydroxide and/or potassium hydroxide.

11. A modular system for performing the process according to any one of claims 1 - 10, comprising:
(a) an absorber having a first inlet for receiving an alkaline solution, a second inlet for receiving a source of carbon dioxide, an outlet for discharging a gas stream depleted in carbon dioxide, and an outlet for discharging a solution comprising carbonate and/or bicarbonate;
(c) a hydrogenation reactor, comprising an inlet for receiving the solution comprising carbonate and/or bicarbonate, an inlet for receiving H₂ gas and an outlet to discharge a solution comprising formate; and
(d) a bipolar membrane electrodialysis reactor, comprising an inlet for receiving the solution comprising formate, an inlet for receiving an electrolyte solution, an outlet for discharging the concentrated formic acid solution and an outlet for discharging a recovered alkaline solution,
wherein the modular system comprises a recycle loop which connects the outlet for discharging the recovered alkaline solution to the inlet for receiving an alkaline solution.

12. The modular system according to claim 11, further comprising one or more modules selected from:
(b) an alkaline water electrolysis reactor, comprising an inlet for receiving the solution comprising carbonate and/or bicarbonate, an outlet for discharging the solution comprising carbonate and/or bicarbonate bicarbonate and one or more outlets for discharging H₂ and O₂; and
(e) a second hydrogenation reactor, comprising an inlet for receiving the concentrated formic acid solution from module (d), an inlet for receiving H₂ gas and an outlet for discharging formaldehyde.
